# EUROPÄISCHE PATENTSCHRIFT

(11) **EP 3 116 564 B1**
(45) Veröffentlichungstag und Bekanntmachung des Hinweises auf die Patenterteilung: **04.09.2019**
(21) Anmeldenummer: 15709514.2
(22) Anmeldetag: 13.03.2015
(51) Int. Cl.: A61M 1/16, A61M 39/12, F16K 7/12

(54) **BLUTBEHANDLUNGSKASSETTE MIT GEDELLTEM FOLIENVENTIL SOWIE BLUTBEHANDLUNGSVORRICHTUNG**
BLOOD TREATMENT CASSETTE HAVING A DENTED FILM VALVE, AND BLOOD TREATMENT DEVICE
CASSETTE DE TRAITEMENT SANGUIN COMPORTANT UNE VALVE À FEUILLE BOSSELÉE ET DISPOSITIF DE TRAITEMENT SANGUIN

(30) Priorität: 14.03.2014 DE 102014103506
(43) Veröffentlichungstag der Anmeldung: 18.01.2017
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Bobbert & Partner Patentanwälte PartmbB
(86) Internationale Anmeldenummer: PCT/EP2015/055320
(87) Internationale Veröffentlichungsnummer: WO 2015/136083

(56) Entgegenhaltungen:
- WO-A1-2010/121819
- WO-A1-2012/175435
- US-A1- 2008 077 068
- US-A1- 2013 331 774

## Beschreibung

Die vorliegende Erfindung betrifft eine Blutbehandlungskassette gemäß dem Oberbegriff des Anspruchs 1 und eine Blutbehandlungsvorrichtung gemäß dem Oberbegriff des Anspruchs 4.

Einmalteilsysteme werden in der Medizin- oder Labortechnik zunehmend als kompakte medizinische Funktionsvorrichtungen wie Kassettensysteme oder Blutbehandlungskassetten ausgeführt, in denen Flüssigkeiten und Gase, insbesondere medizinische Flüssigkeiten und Blut, in Kanälen und Kammern geführt werden. Sind sie zum einmaligen Gebrauch vorgesehen, so spricht man von Kassettendisposables oder Einwegkassetten.

Zumeist handelt es sich hierbei um Hartteil-Folie-Kassetten. Das Hartteil besteht regelmäßig aus einem Spritzgußwerkstoff wie beispielweise PE, PP, PA, ABS, PMMA, PC oder PVC. In ihm sind beispielsweise Schlauchanschlüsse, Konnektoren, Kammern, Kanäle und Zentriereinrichtungen ausgestaltet. Die Kammern und Kanäle sind in der Regel als fluidführende halboffene Strukturen ausgestaltet. Eine Folie aus zum Hartteil kompatiblen Werkstoffen (mit dem Hartteil verschweißbar oder verklebbar) verschließt die halboffenen Strukturen und komplettiert sie zu vollwertigen Kammern und Kanälen. Die Folie kann beispielweise nur an einem äußeren umlaufenden Rand mit der Blutbehandlungskassette verschweißt oder verklebt sein. Ebenfalls gibt es Ausführungen, bei denen die Berandungen der Kammern und Kanäle, die sog. Kanalrandstege, streifenförmig oder flächig mit der Folie verschweißt oder verklebt sind. Auf diese Weise entstehen Blutbehandlungskassetten, die vor dem Einbau in eine Behandlungsmaschine und nach dem Ausbau bereits eine definierte Fluidführung vorgeben.

Bestimmte Bereiche des Hartteils und der Folie werden häufig bewusst nicht miteinander verschweißt oder verklebt. Diese Bereiche können als Folienventile zwischen verschiedenen fluidführenden Bereichen genutzt werden. Zu diesem Zweck wird die Blutbehandlungskassette beim Einsetzen in die Blutbehandlungsvorrichtung zwischen eine Tür und eine Aktor-Sensor-Einheit der Blutbehandlungsvorrichtung eingebaut und anschließend die Tür durch ihr Schließen in eine sog. Verpress-Stellung gebracht, bei der die Folie gegen das Hartteil gepresst und die Blutbehandlungskassette mit der Folie räumlich definiert an die Aktor-Sensor-Matte der Aktor-Sensor-Einheit angekoppelt werden. In die Aktor-Sensor-Matte und Aktor-Sensor-Platte (oder -einheit) integrierte Aktoren (hierin auch als Aktuatoren bezeichnet) können Bewegungen durch die Folie hindurch ausüben, wodurch beispielsweise Pump- oder Ventil-Funktionen realisierbar sind. Mittels wenigstens eines, optional in der Aktor-Sensor-Platte vorgesehenen Sensors können beispielsweise Eigenschaften von Fluiden, welche die Blutbehandlungskassette durchströmen, gemessen werden.

Insbesondere bei Blutbehandlungskassetten mit kanalrandbündig aufgeschweißten Folien und Folienventilen kann es allerdings zu herstelltechnischen oder verfahrenstechnischen Problemen kommen.

Aus der US 2008/077068 A1 sind eine Membranpumpe und verwandte Verfahren bekannt.

Aus der WO 2012/175435 A1 sind Einmalartikel für die Blutbehandlung und Verfahren zum Betrieb derselben bekannt.

In der WO 2010/121819 A1 sind eine externe Funktionseinrichtung, eine Blutbehandlungsvorrichtung zum Aufnehmen einer externen Funktionseinrichtung sowie Verfahren offenbart.

Aus der US 2013/331774 A1 sind medizinische Fluidkassetten, verwandte Systeme und Verfahren bekannt.

Eine erfindungsgemäße Aufgabe kann darin bestehen, eine weitere Blutbehandlungskassette vorzuschlagen. Ferner soll eine Blutbehandlungsvorrichtung zum Verwenden der Blutbehandlungskassette angegeben werden.

Die Aufgabe kann gelöst werden durch eine Blutbehandlungskassette mit den Merkmalen des Anspruchs 1, ferner durch eine Blutbehandlungsvorrichtung mit den Merkmalen des Anspruchs 4.

Erfindungsgemäß wird somit eine Blutbehandlungskassette mit einem als Hartteil ausgestalteten Kassettenkörper und einer Folie vorgeschlagen. Dabei ist die Folie mit dem Hartteil, beispielsweise durch Schweißen oder Kleben, verbunden und das Hartteil ist zumindest abschnittsweise gegenüber einem Äußeren durch die Folie be- oder abgedeckt, so dass aus Hartteil und Folie gemeinsam gebildete Kanäle oder Kammern oder Teile hiervon ausgestaltet werden. Ferner weist das Hartteil wenigstens einen Ventilsitz oder -abschnitt eines Ventils auf, hierin auch als Ventilgrund des Ventils bezeichnet.

Das Ventil ist ausgestaltet, um eine erste und eine hiervon verschiedene, zweite Stellung oder Ventilstellung einzunehmen. Dabei ist die erste Stellung eine geöffnete Stellung, in welcher das Ventil, insbesondere zum Gassterilisieren von Abschnitten des Hartteils, geöffnet ist. In der ersten Stellung berühren sich der Ventilgrund und ein Abschnitt der Folie, welcher im Gebrauch der Behandlungskassette über dem Ventilgrund angeordnet ist oder zu liegen kommt, nicht. Das Ventil ist konfiguriert und vorgesehen, um bei Aufbringen einer - insbesondere in Richtung auf den Ventilgrund hin und mittels einer auf den über dem Ventilgrund angeordneten Abschnitt der Folie wirkenden - Kraft in die zweite, geschlossene Stellung überzugehen. In der zweiten Stellung berühren sich der Ventilgrund und der Abschnitt der Folie, beispielsweise direkt oder indirekt. In der zweiten Stellung ist das Ventil geschlossen.

Des Weiteren weist der Ventilgrund in seiner Längserstreckung einen nicht-geraden Abschnitt auf oder besteht hieraus.

Ferner wird eine erfindungsgemäße Blutbehandlungsvorrichtung vorgeschlagen, welche mit einer Blutbehandlungskassette verbunden oder zur Verbindung hiermit vorgesehen, konfiguriert oder geeignet ist. Sie weist ferner eine Aktor-Sensor-Platte auf. Die Aktor-Sensor-Platte weist wenigstens einen Aktuator auf, welcher wenigstens zwei, vorzugsweise mehr als drei, Teil-Aktuatoren aufweist. Diese sind derart angeordnet, um - vorzugsweise vollständig oder im Wesentlichen - unabhängig voneinander Kraft auf den Abschnitt der Folie des Ventils auszuüben.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Hierin gemachte räumliche Angaben wie "oben", "unten", usw. beziehen sich im Zweifel auf die Darstellung, wie sie den hier beigefügten Figuren zu entnehmen ist.

Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale in beliebiger Kombination aufweisen.

In einigen erfindungsgemäßen, beispielhaften Ausführungsformen ist das Ventil ausgestaltet, um mittels Druckaufbringung eines Aktuators einer Blutbehandlungsvorrichtung auf das Ventil, zu deren Betrieb die Blutbehandlungskassette bestimmungsgemäß mit der Blutbehandlungsvorrichtung verbunden wird, von der ersten Stellung in die zweite Stellung überführbar zu sein.

Erfindungsgemäß ist das Ventil als Phantomventil ausgestaltet.

Die hierin beschriebenen Folienventile werden im Zusammenhang mit der vorliegenden Erfindung auch Phantomventile genannt, da sie im geschlossenen Zustand aus der Sicht der betroffenen Kanäle keine Veränderung des Strömungsraumes im Vergleich zu Kanalstellen oder Kammern ohne Folienventil darstellen. Sie werden mit Blick auf den Strömungsraum - gleich einem Phantom - nicht wahrgenommen.

In einigen erfindungsgemäßen, beispielhaften Ausführungsformen ist der nicht-gerade Abschnitt, hierin auch als gedellter Abschnitt oder Delle bezeichnet, deshalb nicht gerade, weil er unterschiedlichen Abstand zur Folienebene oder zu einer auf der Folie liegenden Ebene oder zu einer Haupterstreckungsebene der Folie oder der Blutbehandlungskassette aufweist (beispielsweise zunächst zunehmenden und anschließend abnehmenden Abstand).

Der nicht-gerade Abschnitt kann konkav zur Folie sein, jedenfalls solange diese eben oder plan liegt.

In bestimmten erfindungsgemäßen, beispielhaften Ausführungsformen ist der nicht-gerade Abschnitt deshalb nicht gerade, weil sein Abstand zur Folienebene oder zu einer auf der Folie liegenden Ebene oder zu einer Haupterstreckungsebene der Folie oder der Blutbehandlungskassette nicht konstant ist.

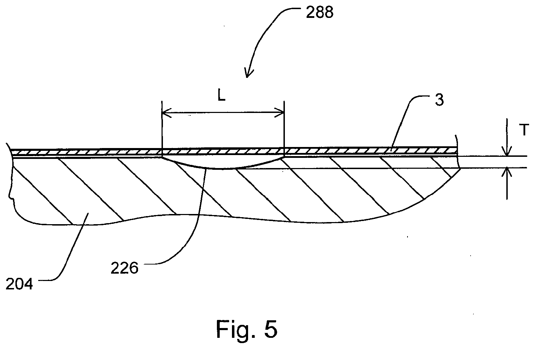

In einigen erfindungsgemäßen, beispielhaften Ausführungsformen ist der nicht-gerade Abschnitt, hierin auch als gedellter Abschnitt oder Delle bezeichnet, deshalb nicht gerade, weil er wie in Fig. 5 gezeigt geformt ist.

Erfindungsgemäß entspricht eine Dellentiefe des Ventilgrunds dem 1- bis 3-fachen der Dicke oder Stärke der Folie. Ferner kann der Ventilgrund hinter benachbarten Kanalrandstegen um das 1- bis 3-fache der Dicke oder Stärke der Folie in Richtung Inneres der Blutbehandlungskassette zurückgesetzt sein.

Erfindungsgemäß ist der Ventilgrund konvex oder konkav (konkav insbesondere zu einer Folienebene oder Haupterstreckungsebene der Folie oder der Blutkassette) ausgestaltet.

In bestimmten erfindungsgemäßen, beispielhaften Ausführungsformen nimmt die Dellentiefe stetig zu und/oder ab.

In bestimmten erfindungsgemäßen, beispielhaften Ausführungsformen weist das Ventil nicht sowohl (oder nicht zugleich) einen Durchlass für eine Hauptströmung als auch einen hiervon unterscheidbaren Durchlass für eine Nebenströmung auf.

In manchen erfindungsgemäßen, beispielhaften Ausführungsformen weist die Aktor-Sensor-Platte eine der Blutbehandlungskassette zugewandte Aktor-Sensor-Matte auf. Diese weist einen Abschnitt auf, welcher dünner ist als benachbarte Abschnitte, und welcher dem Ventilgrund der Blutbehandlungskassette zugewandt ist.

Einige oder alle erfindungsgemäßen Ausführungsformen können einen oder mehrere der oben oder im Folgenden genannten Vorteile aufweisen.

Erfindungsgemäß wird eine technische Lösung vorgeschlagen, welche auf technisch einfache Weise ein zuverlässiges Schließen des Ventils auch bei gegebenen Toleranzen erlaubt.

Zu den weiteren Vorteilen zählt der erfindungsgemäß mögliche Toleranzausgleich, welcher durch die federnde Lagerung des Abstandhalters aber auch durch die mehrteilige oder lamellierte Ausgestaltung des Aktuator zum Schließen des Folienventils möglich ist. Der Aktuator der erfindungsgemäßen Blutbehandlungsvorrichtung dient dabei als Toleranzausgleichvorrichtung für gedellte, also nicht gerade verlaufende Folien-Dichtsitz-Stege, bei denen ein Einbautoleranz- und Formtoleranz-Ausgleich in Richtung längs des Dichtsitz-Steges beispielsweise über unabhängig bewegliche Lamellen gewährleistet wird.

Die Toleranzausgleichvorrichtungen mittels der Aktor-SensorEinheit können dabei vorteilhaft die Dichtwirkung der gedellten Folienventile aufrecht erhalten und sind robust gegenüber Maßtoleranzen der Blutbehandlungskassette, der Blutbehandlungsvorrichtung, oder die Ausrichtung zwischen Blutbehandlungskassette und Blutbehandlungsvorrichtung betreffend. Es scheint überdies, dass ein Strom durch gedellte Folienventile hindurch zuverlässiger bei gedelltem als bei flachem Ventilgrund erfolgen kann. Jedenfalls kann der zum Erzielen einer gewünschten Abdichtung erforderliche technische Aufwand bei der Verwendung gedellter Ventile geringer sein.

Ein weiterer Vorteil gedellter Ventile oder Folienventile kann darin bestehen, dass, gemessen an Folienventilen mit flachem Ventilgrund, bei gegebener, maximal zulässiger Dehnung der Folie - gegenüber dem flachen Ausgangszustand der Folie - beim Öffnen des Ventils bei gleicher Baubreite des Ventils ein ungleich größerer Strömungsquerschnitt erreicht werden kann, welcher beispielsweise das Doppelte betragen kann. Dies beruht auf der erreichbaren Dellentiefe, welche beim Ventil mit gedelltem Ventilsitz ohne Schädigung der Folie erreichbar ist und welche größer als beim flachen Ventilsitz sein kann.

Eine zulässige, maximale Dehnung kann dabei beispielsweise 10 bis 15 %, vorzugsweise 12 %, der Folienlänge über der Erstreckung des Ventilsgrunds oder über der Breite oder Länge der Kassette betragen.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil vereinfachten Figuren gilt:
- Fig. 1: zeigt eine Seitenansicht einer an ihrer Vorderseite mit einer Abdeckungseinrichtung versehenen erfindungsgemäßen Blutbehandlungskassette gemäß einer bevorzugten Ausführungsform;
- Fig. 2: zeigt die externe Funktionseinrichtung der Fig. 1 mit nach zerstörendem Aufschneiden aufgeklappter Abdeckungseinrichtung;
- Fig. 3: zeigt die Blutbehandlungskassette aus Fig. 1 und Fig. 2 von ihrer Rückseite;
- Fig. 4: zeigt in Draufsicht einen Ausschnitt eines Hartteils einer erfindungsgemäßen Kassette;
- Fig. 5: zeigt den in Fig. 4 gezeigten Ventilgrund in Vergrößerung;
- Fig. 6: zeigt einen Schnitt durch die Blutbehandlungskassette der Fig. 4 entlang der Linie A-A mit Blick in Richtung der Pfeile;
- Fig. 7: zeigt die Kassette der Fig. 6, eingelegt in eine Ausführungsform der Blutbehandlungsvorrichtung und von dieser zwischen Tür und Aktor-Sensor-Matte verpresst;
- Fig. 8: zeigt die erfindungsgemäße Blutbehandlungskassette in einer weiteren Ausführungsform, eingelegt in eine Ausführungsform der Blutbehandlungsvorrichtung und von dieser zwischen Tür und Aktor-Sensor-Matte verpresst;
- Fig. 9: zeigt eine Ausführungsform einer in eine erfindungsgemäße Blutbehandlungsvorrichtung eingelegten und von dieser verpressten Blutbehandlungskassette in Draufsicht; und
- Fig. 10: zeigt eine Ausführungsform einer in eine erfindungsgemäße Blutbehandlungsvorrichtung eingelegten und von dieser verpressten Blutbehandlungskassette in Schnittansicht entlang der Linie D-D der Fig. 9.

Die normalen Pfeile in den Figuren zeigen die Richtung des Blutstroms an. Die Blockpfeile zeigen jeweils die Richtung des Substituatstroms an.

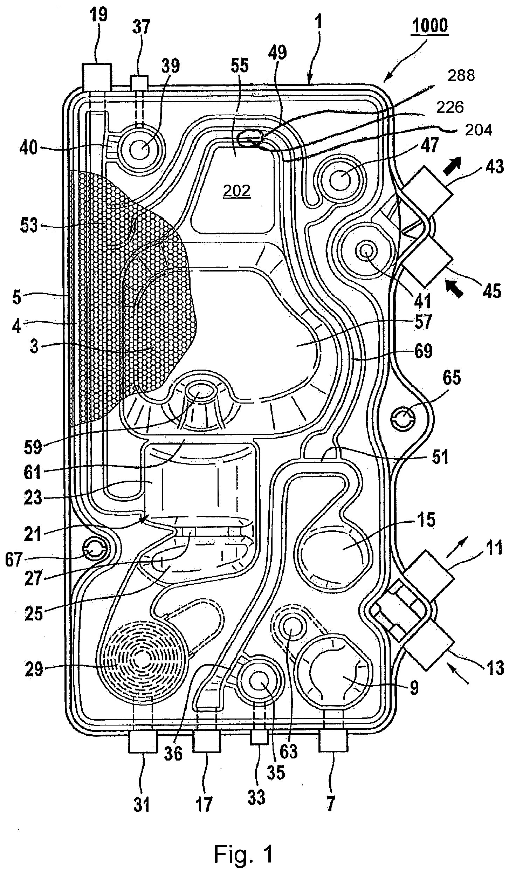

**Fig. 1** zeigt eine Seitenansicht einer erfindungsgemäßen Blutbehandlungskassette 1000, welche an der Oberfläche, auf die in Fig. 1 geblickt wird, mit einer Abdeckungseinrichtung versehen ist.

Die erfindungsgemäße Blutbehandlungskassette 1000 wird im Folgenden auch kurz als Kassette 1000 bezeichnet.

Die Kassette 1000 weist ein Hartteil 1 auf. Wie in Fig. 1 exemplarisch gezeigt, weist das Hartteil 1 Kammern, Kanäle und Ventile auf. Wie in Fig. 1 exemplarisch weiter gezeigt ist, sind die Kammern, Kanäle und Ventile in das Hartteil 1 integriert bzw. zumindest teilweise vom Hartteil 1 ausgestaltet.

Die Kassette 1000 der Fig. 1 ist an ihrer Vorderseite mit einer Abdeckungseinrichtung, hier beispielsweise einer Folie 3, versehen. Die Abdeckungseinrichtung kann eben, d. h. plan, auf das Hartteil 1 aufgeschweißt sein.

Eine Ausgestaltung mit einer dreidimensionalen Ausführung der Schweiß- und Dichtkontur ist erfindungsgemäß ebenfalls möglich.

Die Abdeckungseinrichtung kann die Kammern und/oder Kanäle des Hartteils 1 der Kassette 1000 verschließen, und zwar gegenüber einer dem Hartteil 1 abgewandten Seite der Abdeckungseinrichtung und/oder gegenüber der Atmosphäre.

Wie in Fig. 1 zu erkennen ist, liegt die Folie an einem umlaufenden Dichtsteg 4 auf dem Hartteil 1 der Kassette 1000 auf. Die Folie 3 ist an einer umlaufenden Schweißnaht 5 mit dem Hartteil 1 der Kassette 1000 verschweißt.

Der umlaufende Dichtsteg kann alternativ freiliegend ausgeführt werden.

Die Folie 3 kann an weiteren lokalen Verschweißungen (nicht gezeigt) mit dem Hartteil 1 der Kassette 1000 verbunden sein. Diese können ebenfalls umlaufend, also geschlossen im Sinne einer abschließenden Begrenzung ähnlich einem Ring, und/oder punktförmig sein.

Die Folie 3 kann an lokalen Stellen punkt- oder linienförmig mit dem Hartteil der Kassette 1000 verbunden, z. B. verschweißt, sein, insbesondere an den Randzonen der flüssigkeitsführenden Kanäle.

Die Folie 3 kann durch Laser-Schweißen mit dem Hartteil der Kassette 1000 verbunden werden. Dabei ist es vorteilhaft, wenn der lokale Hitzeeintrag unter Verwendung einer Licht absorbierenden Komponente erfolgt. Die Licht absorbierende Komponente kann Bestandteil des Materials der Folie und/oder des Hartteils sein oder eine Schicht, die zwischen Folie und Hartteil oder über der Folie angeordnet ist. Die Schicht kann eine Folienschicht sein.

Die Kassette 1000 kann wenigstens mit der in Fig. 1 gezeigten Vorderseite an eine Blutbehandlungsvorrichtung (in Fig. 1 nicht gezeigt) angekoppelt werden. Eine beispielhafte Vorgehensweise zum geeigneten Ankoppeln einer Kassette 1 an eine Ankoppelfläche einer Blutbehandlungsvorrichtung ist in den Patentanmeldungen 10 2009 012 633.3 mit dem Titel *"Vorrichtung zum Verbinden einer externen Funktionseinrichtung mit einer Anordnung, Anordnung aufweisend eine solche Vorrichtung und Verfahren zum Verbinden",* die am 10. März 2009 beim DPMA eingereicht wurde, und 10 2009 012 632.5 mit dem Titel *"Abdichtungseinrichtung zum Abdichten eines Volumens einer medizinischen Behandlungsanordnung gegen ein weiteres Volumen sowie Anordnung und Verfahren",* die ebenfalls am 10. März 2009 beim DPMA eingereicht wurde, beschrieben.

Die Kassette 1000 kann mit der Ebene der Folie 3 - oder über diese - an eine Ankoppelfläche der Blutbehandlungsvorrichtung angekoppelt werden. Die Ankoppelfläche kann vorzugsweise dreidimensional ausgeführt sein.

Die Ankoppelfläche der Blutbehandlungsvorrichtung kann zum Beispiel an einem in Fig. 1 oberen Abschnitt hiervon um 8° gegen eine in Fig. 1 von oben nach unten verlaufende Senkrechte nach hinten (in der Fig. 1 die sich vom Betrachter in die Zeichenebene hinein erstreckende Richtung) geneigt sein.

Die Kassette 1000 weist einen arteriellen Patientenanschluss 7 auf.

Die Kassette 1000 weist eine arterielle Druckmesskammer 9 auf. Diese kann entsprechende Sensoren aufweisen. Die Sensoren können bevorzugt über eine Verkabelung Signale übermitteln. Die Sensoren können aber auch so ausgeführt sein, dass sie kabellos Signale übermitteln.

Die Kassette 1000 weist einen Konnektor 11 für den Blutaustritt aus der Kassette 1000 sowie einen Konnektor 13 für den Bluteintritt in die Kassette 1000 auf.
Die beiden Konnektoren 11 und 13 sind mit einem Pumpschlauchsegment oder -set einer Blutpumpe verbindbar.

Die Kassette 1000 weist ferner eine Kammer 15 mit einer Druckmessstelle zur Druckmessung im extrakorporalen Blutkreislauf vor dem Dialysator ("Prä-Filter") bzw. nach der Pumpe ("Post-Pumpe") auf.

An der Kammer 15 kann durch die Folie 3 hindurch bzw. über die Folie 3 der Druck im extrakorporalen Kreislauf vor dem Dialysator gemessen werden.

Die Kassette 1000 weist eine arterielle Filterleitung 17 sowie eine venöse Filterleitung 19 auf.

Das Innere der Kassette 1000 weist eine venöse Blutkammer 21 auf. Die venöse Blutkammer 21 ist in einen oberen Raum 23 und einen unteren Raum 25 unterteilt.

Der obere Raum 23 der venösen Blutkammer 21 kann eine seitlich tangentiale Bluteinströmung zulassen. Dabei kann Blut seitlich durch den Einlass (der linken Seite in Fig. 1) in den oberen Raum 23 einströmen und sich tangential zu den Wänden des oberen Raums 23 ausbreiten. Eine seitlich tangentiale Bluteinströmung kann eine Zone mit im Wesentlichen oder vollständig stabiler Rotationsströmung des Bluts in dem oberen Raum 23 der venösen Blutkammer 21 erzeugen.

Der untere Raum 25 der venösen Blutkammer 21 kann eine Beruhigungszone für die Blutströmung darstellen. Es ist möglich, dass in einer derartigen Beruhigungszone im Wesentlichen keine oder überhaupt keine Rotationsströmung des Bluts vorliegt.

Die venöse Blutkammer 21 ist durch eine Querschnittsverjüngung 27 des Hartteils 1 der Kassette 1000 in den oberen Raum 23 und den unteren Raum 25 aufgeteilt. Die Querschnittsverjüngung 27 reduziert den Querschnitt der venösen Blutkammer 21 derart in seiner Breite und Tiefe, dass sich eine Strömungsschnelle ergibt, nach deren Durchquerung ein die venöse Blutkammer 21 der Kassette 1000 durchströmendes Fluid eine langsamere Strömungsgeschwindigkeit annimmt. Der obere Raum 23 und der untere Raum 25 stehen in Fluidkommunikation.

Durch eine derartige Konstruktion, d. h. einer Aufteilung der venösen Blutkammer 21 in eine Zone mit im Wesentlichen oder vollständig stabiler Rotationsströmung des Bluts und in eine Beruhigungszone für die Blutströmung, kann vorteilhaft eine effiziente Abscheidung für Luft aus dem Blut oder Fluid erreicht werden.

Wandungen des oberen Raums 23 und des unteren Raums 25 der venösen Blutkammer 21 können in geeigneter Weise einer Neigung des oberen Abschnitts der Kassette 1000 in Fig. 1 gegen die Vertikale, beispielsweise einer Neigung des oberen Teils der in Fig. 1 gezeigten Kassette 1000 um 8° nach hinten (in die Zeichenebene hinein), angepasst werden. Sie können in geeigneter Weise derart gerundet ausgestaltet sein, dass sie vorteilhaft eine strömungsoptimierte Berührfläche für Fluide darstellt, welche die venöse Blutkammer 21 durchströmen.

Die Kassette 1000 weist einen Gerinnselfänger 29 auf.

Als Gerinnselfänger kann vorzugsweise ein Gerinnselfänger verwendet werden, wie er in der von der Anmelderin der vorliegenden Erfindung in der Patentanmeldung 10 2009 024 495.6 mit dem Titel *"Gerinnselfänger, externe Funktionseinrichtung, Blutkreislauf sowie Behandlungsvorrichtung",* die am 10. Juni 2009 beim DPMA eingereicht wurde, offenbart wurde.

Am Gerinnselfänger 29 kann durch die Folie 3 hindurch bzw. über die Folie 3 der Druck im extrakorporalen Kreislauf gemessen werden, also insbesondere nach Durchlaufen des Dialysators.

Die Kassette 1000 weist einen venösen Patientenanschluss 31 auf.

Die Kassette 1000 weist eine arterielle Heparin-Zugabestelle 33 auf. Dabei gilt zu beachten, dass die Heparin-Zugabestelle 33 (wie auch eine venöse Heparin-Zugabestelle 37) auch zur Zugabe anderer pharmakologischer Wirkstoffe als Heparin, welche nur vorzugsweise Antikoagulantien sind, oder Wirkstoffkombinationen geeignet und vorgesehen sein kann. Dies ist stets auch dann zu beachten, wenn zuvor oder im Folgenden von Heparin in beliebigem Zusammenhang die Rede ist.

Die Kassette 1000 weist ein Rückschlagventil 35 der arteriellen Heparin-Zugabestelle 33 auf.

Beispielhafte Rückschlagventile zum Einsatz als Rückschlagventil 35 der arteriellen Heparin-Zugabestelle 33 sowie als weitere Rückschlagventile der Kassette 1000 sind in der Patentanmeldung 10 2009 024 469.7 der Anmelderin der vorliegenden Erfindung mit dem Titel *"Ventilvorrichtung, Ventileinsatz, externe Funktionseinrichtung, Behandlungsvorrichtung sowie Verfahren",* die am 10. Juni 2009 beim DPMA eingereicht wurde, offenbart.

Die Kassette 1000 weist ein arterielles Heparin-Zugabeventil 36 auf. Mit Hilfe des arteriellen Heparin-Zugabeventils 36 kann die Zugabe von Heparin in die arterielle Filterleitung 17 gesteuert oder geregelt werden.

Das arterielle Heparin-Zugabeventil 36 kann als so genanntes Phantomventil ausgestaltet sein.

Der Begriff "Phantomventil", wie er hierin verwendet wird, bezeichnet ein Element mit einer mittels Aktors erreichbaren Aktor-Fläche (hier beispielsweise eine Aktor-Membran), welches die Funktion eines Ventils übernehmen kann.

Die Aktor-Membran ist unter Aufbringung einer Kraft hierauf, z. B. einer Druckkraft, in eine Richtung bewegbar, ausdehnbar bzw. wölbbar oder dergleichen. Durch das Bewegen oder Ausdehnen der Aktor-Membran kann sich diese an ein Element, wie eine Abdichtungseinrichtung, etwa einen Steg, anlegen oder von diesem entfernen. Die Aktor-Membran kann somit beispielsweise eine Abdichtung bewirken bzw. verstärken oder beenden bzw. verringern.

Wenn die Kraft wieder von der Aktor-Membran genommen wird, kann diese in beispielsweise eine Grundposition, z. B. einen nicht gewölbten Zustand, zurückkehren.

Ein Phantomventil zur Verwendung als arterielles Heparin-Zugabeventil 36 sowie weitere Phantomventile der Kassette 1000 können mit oder aus einem Stegabschnitt eines Kanals am Hartteil 1 der Kassette 1000 und einem dem Stegabschnitt an- oder gegenüberliegenden Abschnitt der Folie 3 ausgestaltet werden.

Phantomventile können durch Aktoren der Blutbehandlungsvorrichtung betätigt werden.

Zum Schließen eines Phantomventils kann der Abschnitt der Folie 3 auf den Stegabschnitt gedrückt werden. Zum Öffnen des Phantomventils kann der Abschnitt der Folie 3 wieder vom Stegabschnitt abgehoben werden.

Weitere Beispiele und/oder Ausführungsformen für Phantomventile können der Patentanmeldung 10 2009 012 632.5 mit dem Titel *"Abdichtungseinrichtung zum Abdichten eines Volumens einer medizinischen Behandlungsanordnung gegen ein weiteres Volumen sowie Anordnung und Verfahren",* die am 10. März 2009 von der vorliegenden Anmelderin beim DPMA eingereicht wurde, sowie der DE 100 53 441 A1 und der DE 102 24 750 A1 entnommen werden.

Die Kassette 1000 weist eine venöse Heparin-Zugabestelle 37 auf. Die venöse Heparin-Zugabestelle 37 kann als Luer-Konnektor ausgestaltet sein.

Die Kassette 1000 weist ein Rückschlagventil 39 der venösen Heparin-Zugabestelle 37 auf.

Die Kassette 1000 weist ein venöses Heparin-Zugabeventil 40 auf. Mit Hilfe des venösen Heparin-Zugabeventils 40 kann die Zugabe von Heparin in die venöse Filterleitung 19 gesteuert oder geregelt werden.

Die Kassette 1000 weist eine Substituat-Zugabestelle 41 bzw. einen Substituatkonnektor auf.

Die Substituat-Zugabestelle 41 kann eine Verbindungseinrichtung sein, wie sie in der Patentanmeldung 10 2009 024 575.8 der vorliegenden Anmelderin beschrieben ist, welche mit dem Titel *"Verbindungseinrichtung und Verfahren zum Konnektieren wenigstens zweier fluidführender medizintechnischer Systeme, sowie medizintechnische Vorrichtung"* am 10. Juni 2009 von der vorliegenden Anmelderin beim DPMA eingereicht wurde.

Die Substituat-Zugabestelle 41 kann mit einem Berührschutzelement (nicht gezeigt) versehen sein. Die Substituat-Zugabestelle 41 kann mit einem Tropfschutz (nicht gezeigt) versehen sein. Der Tropfschutz kann durch eine integrierte Verschlusshülse realisiert werden. Der Tropfschutz kann ein Heraustropfen von Resten von Substituat und/oder Blut beim Lösen der Kassette 1000 und anschließendem Entnehmen der Kassette 1000 aus der Blutbehandlungsvorrichtung verhindern.

Der Tropfschutz kann abnehmbar ausgeführt sein. Er kann als Haube oder Deckel ausgestaltet sein.

Die Substituat-Zugabestelle 41 oder ein anderer Abschnitt der Kassette 1000 kann ferner einen Originalitätsschutz bieten, durch welchen der Anwender ohne Mühe oder auf einen Blick erkennt, ob die Kassette 1000 bereits benutzt wurde. Dieser Originalitätsschutz kann mittels des Berührschutzelements, der Verschlusshülse oder einer anderen Struktur realisiert sein. Die entsprechende Struktur kann vorzugsweise ihre Position innerhalb der oder bezogen auf die Kassette 1000 erkennbar verändern. Sie kann vorzugsweise ihre Gestalt verändern.

Zudem kann die Substituat-Zugabestelle 41 oder ein anderer Abschnitt der Kassette 1000 einen Wiederverwendungsschutz bzw. Schutz gegen ein Wiederverwenden bieten. Vorzugsweise durch eine Verschlusshülse wird die Kassette 1000 - vorzugsweise irreversibel - im Hinblick auf den Versuch einer Wiederverwendung unbrauchbar gemacht. Soll die Kassette 1000 trotzdem erneut verwendet werden wollen, so messen Sensoren der Blutbehandlungsvorrichtung nicht die Signalverläufe, die bei Verwenden einer neuen Kassette gemessen werden würden. Dies kann hierauf beruhen, dass keine Flüssigkeit in die Kassette 1000 oder in die Substituat-Zugabestelle 41 gelangen kann oder wenigstens nicht in ausreichender oder üblicher Menge. Die Steuereinheit der Blutbehandlungsvorrichtung kann dies erkennen. Es kann eine Warnung veranlasst werden.

Als Originalitätsschutz oder Wiederverwendungsschutz kann vorzugsweise ein Originalitätsschutz oder ein Wiederverwendungsschutz verwendet werden, wie er in der von der Anmelderin der vorliegenden Erfindung in der Patentanmeldung 10 2009 024 575.8 mit dem Titel *"Verbindungseinrichtung und Verfahren zum Konnektieren wenigstens zweier fluidführender medizintechnischer Systeme, sowie medizintechnische Vorrichtung",* die am 10. Juni 2009 beim DPMA eingereicht wurde, offenbart wurde.

Die Kassette weist einen Konnektor 43 für einen Substituataustritt aus der Kassette 1000 sowie einen Konnektor 45 für einen Sustituateintritt in die Kassette 1000 auf.

Die Konnektoren 43 und 45 sind mit einem Pumpschlauchsegment oder -set einer Substituatpumpe verbindbar.

Die Kassette 1000 weist ein Rückschlagventil 47 für Substituatzugabe auf.

Über Betätigung des Rückschlagventils 47 kann Substituat in eine Substituatleitung 49 eingebracht werden.

Die Kassette 1000 weist ein Prädilutions-Zugabeventil 51 auf. Das Prädilutions-Zugabeventil 51 kann als Phantomventil ausgestaltet sein.

Die Kassette 1000 weist ein Postdilutions-Zugabeventil 53 auf. Das Postdilutions-Zugabeventil 53 kann als Phantomventil ausgestaltet sein.

Die Kassette 1000 weist eine Single-Needle-Sterilmembran 55 auf.

Die Kassette 1000 weist eine Single-Needle-Kammer 57 auf. Die Single-Needle-Kammer 57 ist in Fig. 1 oberhalb der venösen Blutkammer 21 angeordnet.

Im Inneren der Single-Needle-Kammer 57 ist ein Blutschwallumleitungselement 59 angeordnet. Das Blutschwallumleitungselement 59 kann dem Abbremsen eines Blutschwalls und/oder dem Auslöschen dessen Impulses dienen.

Eine Verbindung mit einem Inneren der Single-Needle-Kammer 57 kann mittels einer Verbindungseinrichtung vorgesehen sein, wie sie in der von der Anmelderin der vorliegenden Erfindung mit dem Titel *"Einrichtung sowie externe Funktionseinrichtung und Behandlungsvorrichtung zum Behandeln von medizinischen Fluiden"* unter 10 2009 024 467.0 am 10. Juni 2009 beim DPMA eingereichten Patentanmeldung offenbart wurde.

Die Kassette 1000 weist ein Single-Needle-Blutventil 61 auf. Das Single-Needle-Blutventil 61 kann als Phantomventil ausgestaltet sein.

Die Kassette 1000 weist eine Absaugstelle 63 auf. Die Absaugstelle 63 kann zur Vakuumankopplung der Kassette 1000 an die Blutbehandlungsvorrichtung dienen, wie dies beispielsweise in der Patentanmeldung DE 10 2007 042 964 A1 mit dem Titel *"Vorrichtung und Verfahren zur Behandlung einer medizinischen Flüssigkeit",* die am 10. September 2007 beim DPMA eingereicht wurde, beschrieben ist.

Die Kassette 1000 weist ein primäres Ausrichtungszentrum 65 auf. Das primäre Ausrichtungszentrum 65 kann vorteilhaft zum Ausrichten und/oder Verrasten der Kassette 1000 an der Blutbehandlungsvorrichtung dienen.

Die Kassette 1000 weist eine sekundäre Ausrichtungsstelle 67 auf. Die sekundäre Ausrichtungsstelle 67 kann zum Ausrichten und/oder Verrasten der Kassette 1000 an der Blutbehandlungsvorrichtung dienen.

Die Kassette 1000 ist vor Beginn des Primens mit Gas (z. B. steriler Luft) gefüllt. Beim Primen des extrakorporalen Blutkreislaufs muss diese Gasfüllung verdrängt werden. Eine Blutbehandlungskassette stellt hierbei allgemein eine besondere Herausforderung dar, weil es sowohl aufsteigende als auch fallende Leitungen und zudem Kammern gibt, in denen keine "Luftnester" verbleiben dürfen. Die vorliegende Kassette 1000 weist zu diesem Zweck besondere Konstruktionsmerkmale auf:
Die Kammer 15 zum Messen des arteriellen Drucks ist derart konstruiert, dass die gesamte Luft in ein Pumpschlauchsegment aufsteigen kann. Es gibt hierbei vorteilhaft keine Toträume. Luft, die aus der arteriellen Druckmesskammer in das Pumpschlauchsegment der Blutpumpe selbständig aufsteigt, wird ab dem Eingriffsbereich Blutpumpe (z. B. durch die Rollen einer Rollenpumpe) durch das Pumpschlauchsegment zwangsgefördert. Sobald die Pumpe keinen Einfluss mehr hat (weil z. B. die Rollen in Ausgriff gehen) steigt die Luft selbsttätig in Förderrichtung in die Kassette 1000 auf.

Die venöse Rückführleitung (bzw. ein venöser Abschnitt des extrakorporalen Kreislaufs) ist eine Fallleitung. Ab einem gewissen darin herrschenden Volumenstrom (z. B. 200 ml/min bei der in Fig. 1 gezeigten Kassette 1000) werden Luftblasen im Blut auch entgegen der Erdbeschleunigung "mitgerissen". Dieser Effekt wird bei den Fallleitungen ausgenutzt. Die Leitungsquerschnitte der Fallleitungen sind so klein ausgelegt, dass durch die Strömungsgeschwindigkeit eine Zwangsförderung der Luftblasen auch entgegen der Erdbeschleunigung funktioniert.

In der venösen Blutkammer 21 sind große Querschnitte vorgesehen, derart, dass aufgrund der dort langsamen bzw. niedrigen Strömungsgeschwindigkeiten Luftblasen verlässlich entgegen der Hauptströmungsrichtung aufsteigen können.

Weitere konstruktive Besonderheiten der Kassette 1000 sind:
Die Phantomventile 40, 51 und 53 sind räumlich derart ausgerichtet, dass Blut (welches eine höhere Dichte als Wasser bzw. Substituat etc. hat) bei Betrieb der Kassette 1000 mit Blut kaum "nach oben" oder "zur Seite" in geöffnete Phantomventile eindringen kann, weil es gegenüber dem leichteren Wasser absinkt. Eine solch vorteilhafte Ausrichtung ist durch die Phantomventile 40, 51, und 53 realisiert. Beim Ventil 36 besteht hingegen kein solches Erfordernis, d. h. dort kommt es auf die Orientierung nicht an.

Der Leitungskanal (Stichkanal) unter dem Rückschlagventil 47 für Substituatzugabe ist aus demselben Grunde aufsteigend konstruiert. Im Falle einer Fehlfunktion der Prä- und/oder Postdilutionsventile 51 und 53 und einer daraus resultierenden Blut-Bypassströmung kann kein Blut in die Substituatleitung 49 aufsteigen. Vielmehr strömt das Blut an der Mündung der entsprechenden Stichleitung vorbei.

Die Neigung der Kassette 1000 beträgt vorzugsweise 5° bis 11°, besonders bevorzugt die bereits genannten 8°.

Das Bezugszeichen 288 bezeichnet ein Phantomventil, welches in einer ersten Stellung einen Einstrom in die Kammer 202 ermöglicht oder in einer zweiten Stellung unterbindet. In seiner einfachsten, von der vorliegenden Erfindung umfassten Ausgestaltung ist ein Phantomventil ein Folienventil, bei welchem ein Fluidweg zwischen Hartteil 1 und darüberliegendem Abschnitt der Folie 3 durch vorübergehendes Anpressen der Folie 3 auf einen Ventilgrund, etwa einen Steg oder anderen Abschnitt des Hartteils 1, unterbunden und nach Aufheben der anpressenden Kraft wieder geöffnet wird.

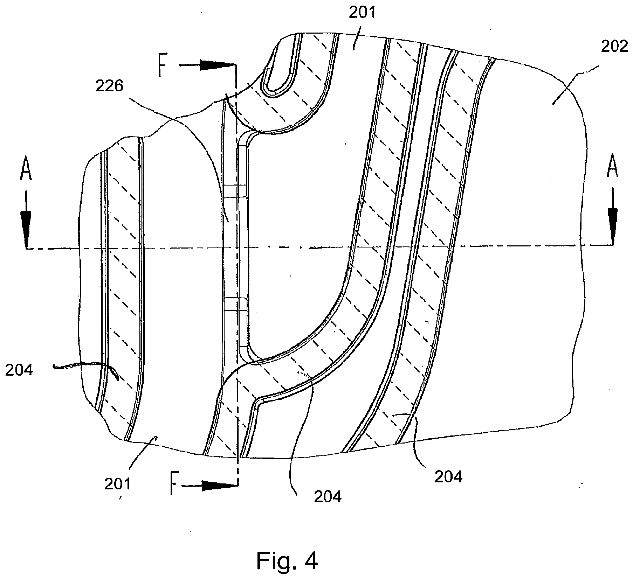

Die Bezugszeichen 202, 204 und 226 werden mit der Beschreibung der Fig. 4 bis 10 erläutert.

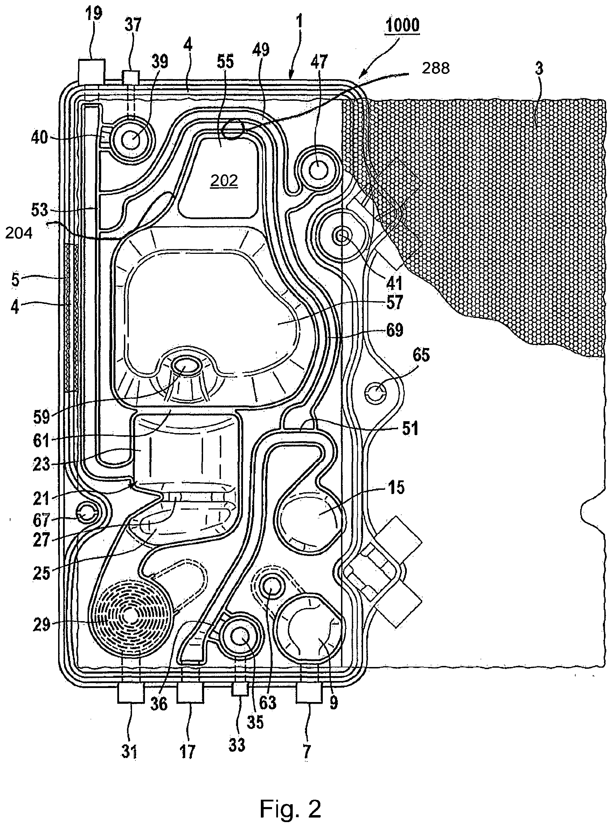

**Fig. 2** zeigt die Kassette 1000 der Fig. 1, wobei die Folie 3 am linken Rand der Kassette 1000 sowie oben und unten zur besseren Illustration zerstörend aufgeschnitten und nach rechts aufgeklappt zu erkennen ist.

Wie in Fig. 2 gezeigt, weist die Folie 3 eine Oberflächenstrukturierung auf.

Fig. 2 zeigt die nach Aufschneiden der Folie 3 detaillierter zu erkennenden Elemente im Inneren der Kassette 1000.

Zur Vermeidung von Wiederholungen wird auf die vorstehend bei der Beschreibung der Fig. 1 erörterten Ausgestaltungen der einzelnen Elemente verwiesen.

Gut zu erkennen ist hierbei, dass die Kassette 1000 einen Dichtsteg 69 aufweist. Der Dichtsteg 69 kann zum Beispiel zum Ausgestalten des Prädilutions-Zugabeventils 51 eingesetzt werden.

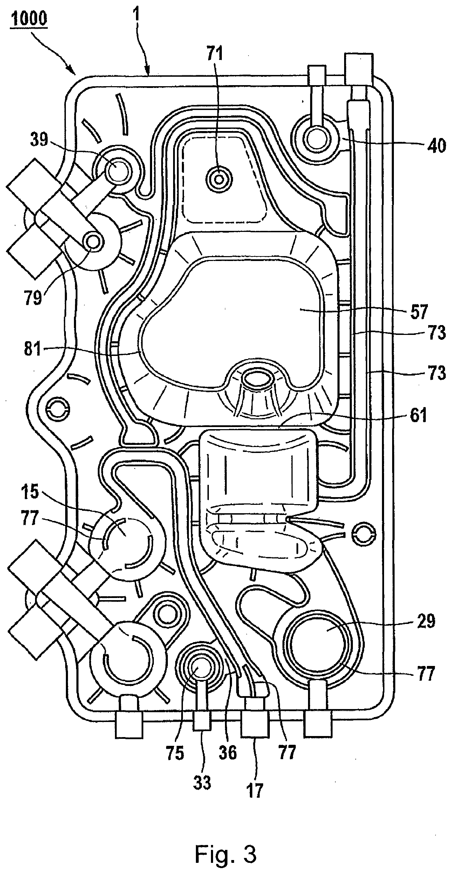

**Fig. 3** zeigt die Kassette 1000 von ihrer Rückseite. Ist die Kassette 1000 an die Blutbehandlungsvorrichtung angekoppelt, so wird ein Betrachter beim Öffnen einer Tür der Blutbehandlungsvorrichtung zum Entnehmen der Kassette 1000 auf diese Rückseite blicken.

Die Kassette 1000 weist einen Single-Needle-Luft-Konnektor 71 auf. Es kann vorgesehen sein, geräteseitig und/oder blutseitig ein Abstützgitter (nicht gezeigt) der Single-Needle-Sterilmembran 55 am Single-Needle-Luft-Konnektor 71 anzuordnen.

Die Kassette 1000 weist mehrere Stützstege auf. Die Stützstege weisen von einander verschiedene Höhen, bezogen beispielsweise auf die Ebene der Folie 3, auf. Die Stützstege erheben sich in der dem Betrachter in Fig. 3 zugewandten Seite der Kassette 1000, also aus der Zeichenebene der Fig. 3 heraus.

Die Kassette 1000 weist Stützstege 73 mit einer Höhe von 5 mm, Stützstege 75 mit einer Höhe von 8 mm, Stützstege 77 mit einer Höhe von 13 mm, Stützstege 79 mit einer Höhe von 24 mm und Stützstege 81 mit einer Höhe von 31 mm auf. Die vorstehenden und andere Zahlenwerte sind natürlich rein exemplarisch zu verstehen.

Die Stützstege können dazu dienen, die Kassette im angekoppelten Zustand an eine Blutbehandlungsvorrichtung gegen einen Deckel einer Aufnahmeeinrichtung der Blutbehandlungsvorrichtung zum Aufnehmen der Kassette abzustützen. Beispielhafte Ausführungsformen einer derartigen Ankopplung der Kassette an die Blutbehandlungsvorrichtung sind in der Patentanmeldung 10 2009 012 633.3 mit dem Titel *"Vorrichtung zum Verbinden einer externen Funktionseinrichtung mit einer Anordnung, Anordnung aufweisend eine solche Vorrichtung und Verfahren zum Verbinden",* die am 10. März 2009 beim DPMA eingereicht wurde, angegeben.

In Fig. 3 ist die Kassette 1000 so gezeigt, wie der Anwender/Betrachter nach ihrem Ankoppeln an das Maschineninterface auf sie blickt. Die Neigung der Kassette 1000 zur Maschine ist "nach hinten geneigt" ausgeführt, so dass die obere Kante weiter vom Anwender/Betrachter entfernt ist als die untere Kante.

Die nach oben zeigenden Flächen der venösen Blutkammer 21 und der Single-Needle-Kammer 57 weisen dementsprechend eine Neigung derart auf, dass Luftblasen trotz der Neigung der Kassette 1000 noch zuverlässig an der Innenseite aufsteigen können. Grundsätzlich ist alternativ natürlich auch ein Kassettendesign möglich, das keine Neigung der Kassette vorsieht.

Die folgenden Figuren zeigen Abschnitte einer erfindungsgemäßen Kassette 1000, welche in allen Merkmalen mit jener Kassette 1000 der Fig. 1 bis 3 übereinstimmen kann, aber nicht muss, soweit sie nicht in den im Folgenden erläuterten Ausführungsformen hiervon abweicht. Jedenfalls kann die erfindungsgemäße Kassette 1000 der folgenden Figuren beliebige Merkmale der in Fig. 1 bis Fig. 3 gezeigten Kassette 1000 aufweisen, sofern die jeweilige Merkmalskombination vom Fachmann nicht als technisch unmöglich erkannt wird.

**Fig. 4** zeigt in Draufsicht einen Ausschnitt eines Hartteils 1 einer Kassette 1000, beispielsweise jener der vorangegangenen Figuren. Das Hartteil 1 hat Kanäle 201, eine Kammer 202, umlaufende ebene Kanalrandstege 204, an welchen die Folie 3 mit dem Hartteil 1 verklebt oder verschweißt ist. Dargestellt ist ferner ein gedellt ausgeführter Abschnitt 226 des Hartteils 1, welcher sich in die Kanalrandstege 204 fortsetzt oder diese unterbricht. Die Folie 3 ist nicht mit dem darunter liegenden Abschnitt 226 des Hartteils 1 verklebt oder verschweißt, was die oben beschriebene Ventilwirkung ermöglicht. Der Abschnitt 226 wird hierin auch als Foliendichtsitz-Steg 226 oder Ventilgrund 226 bezeichnet.

**Fig. 5** zeigt den in Fig. 4 gezeigten Ventilgrund 226 in Vergrößerung. Dieser weist eine Dellentiefe T, eine Dellenbreite und eine Ventilgrund- oder Dichtsitzsteg-Länge L auf. Die nicht darstellbare Dellenbreite erstreckt sich in die Zeichenebene hinein. Der Ventilgrund 226 ist konkav insbesondere zur Ebene, in welcher sich die Folie 3 erstreckt. Der Ventilgrund 226 ist aufgrund der Dellenform oder konkaven Form ein nicht-gerader Abschnitt im Sinne der vorliegenden Erfindung.

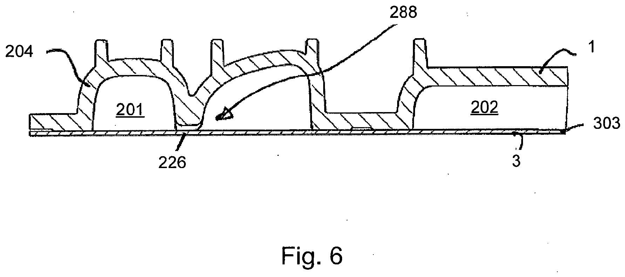

**Fig. 6** zeigt einen Schnitt durch die Kassette 1000 der Fig. 4 entlang der Linie A-A mit Blick in Richtung der Pfeile.

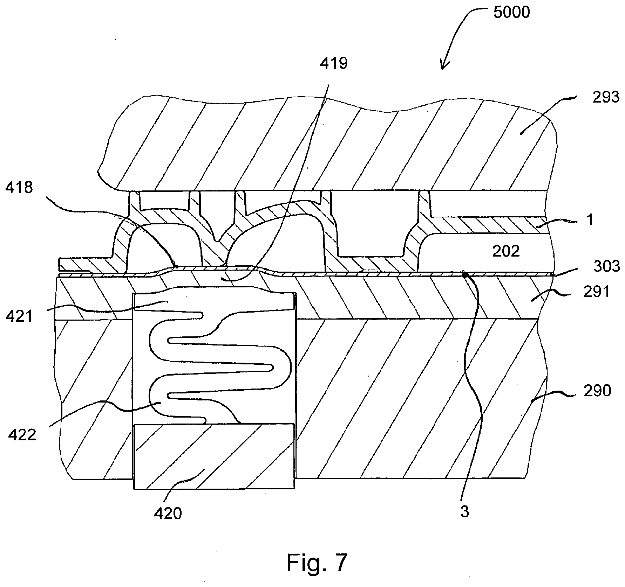

**Fig. 7** zeigt die Kassette 1000 der Fig. 6, eingelegt in eine Ausführungsform einer Blutbehandlungsvorrichtung 5000 und von dieser zwischen einer Tür 293 und einer Aktor-Sensor-Matte 291 verpresst. Die Kassette 1000 ist damit im aufgerüsteten, betriebsfertigen Zustand gezeigt, und zwar in Ausschnitten von der Seite mit (bezogen auf das Zeichenblatt der Fig. 7) horizontal ausgerichteter Folienebene oder Haupterstreckungsebene 303 der Verbindung zwischen Hartteil 1 und Folie 3.

In der beispielhaften Ausführungsform der Fig. 7 fährt ein zum Folienventil-Dichtsitz passender Aktuator 420 der Aktor-Sensor-Platte 290 mit seinem vorderen Abschnitt in einen dem Dichtsitz zugeordneten Abschnitt 419 der Aktor-Sensor-Matte 291 ein, welcher dünner als benachbarte Abschnitte der Aktor-Sensor-Matte 291 ist oder eine verminderte Wandstärke aufweist. Der dünnere Abschnitt 418 wirft, wenn mittels Aktuator betätigt, die Aktor-Sensor-Matte 291 zu einer Erhebung 418 auf, welche das Ventil 288 verschließt (also in die zweite Stellung überführt).

Der zum Schließen des Ventils 288 vorgesehene Aktuator kann beispielsweise der in Fig. 7 gezeigte Anpressstempel sein, welcher in Kontakt mit dem dünneren Abschnitt 419 der Aktor-Sensor- Matte 291 steht.

Der Anpressstempel der Fig. 7 kann optional lamelliert sein oder mehrere Teil- oder Lamellenstempel aufweisen, welche getrennt voneinander auf die Folie 3 einwirken können.

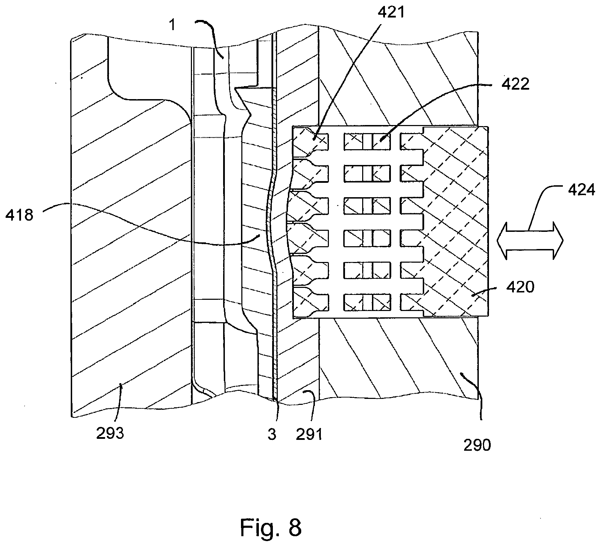

So weist der Anpressstempel der **Fig. 8** beispielweise einen oder mehrere Lamellenstempel 421, eine oder mehrere Lamellenfedern 422 und einen oder mehrere verschiebliche oder fest eingebaute Stempelkörper 420 auf oder besteht hieraus. Der dargestellte Anpressstempel ist beispielsweise ein faserverstärkter Thermoplast. Letzterer kann vorteilhaft preisgünstig gefertigt werden.

Zum Toleranzausgleich in X-Richtung, welche der Horizontalen in Fig. 8 entspricht, zwischen Kassette 1000 und Blutbehandlungsvorrichtung 5000 erstreckt sich die Erhebung oder Ausbeulung 418 der Aktor-Sensor-Matte 291 auf der segmentierten Stirnseite des Anpressstempels prismatisch in an den Ventilgrund 226 angepasster Form in X-Richtung nach links und rechts von der Ventildichtsitzkante 226.

Zum Toleranzausgleich in die Y-Richtung, welche der Vertikalen in Fig. 8 entspricht, kann vorteilhaft eine Segmentierung des Aktuators in unabhängige, einzeln federnd nachgiebige Lamellenstempel erfolgen.

Die Lamellierung des Anpressstempels kann ein Toleranzproblem bei gedellten Folienventilen verhindern, welches zu einer toleranzbedingten Verschiebung zwischen Kassette und Blutbehandlungsvorrichtung in Y-Richtung zu einer deutlichen geometrischen Fehlanpassung in Z-Richtung zwischen der Ventildichtsitzdelle 226 im Hartteil 1 und der vorgeformten Ausbeulung in der Aktor-Sensor-Matte 291 führen kann. Dies kann zur Folge haben, dass entlang der Dichtsitzkante 226 jeweils auf der einen Seite bezüglich der Ventilmitte unverpresste Hohlräume und auf der anderen Seite höherverpresste Bereiche entstehen, die die vorgeformte Ausbeulung der Aktor-Sensor-Matte 291 von der optimalen Formgebung abweichen lassen. Auf diese Weise könnte eine gleichmäßig verpresste und lückenlose Anschmiegung der Folie 3 an die gedellte Ventildichtsitzkante 226 aufgehoben und das Ventil 288 undicht werden. Dies kann mittels der hierin beschriebenen Lamellierung (auch als Segmentierung zu verstehen) vorteilhaft verhindert werden.

Aufgrund der Lamellierung oder Segmentierung passen sich die einzelnen Lamellenstempel, welche unter annähernd gleichbleibender Verpresskraft (abhängig von der Federkennlinie der Lamellenfedern) in Z-Richtung ein- und ausfedern können, wodurch sie sich einzeln an die in Y-Richtung verschobene Delle der Kassette 1000 anpassen und die Form und Lage der Erhebung 418 ausreichend genau übernehmen, an. Damit wird Ventildichtheit auch unter dem Einfluss schwankender Gesamt-Verpresskräfte und bei lokalen Abnützungen und Relaxationen der Aktor-Sensor-Matte 291 erzielt.

Die hier beispielhaft dargestellte einstückige Ausführung des lamellierten Anpressstempels ist besonders vorteilhaft, weil hier keine Gleitlagerungen einzelner Segmente vorliegen, welche wegen der damit verbundenen Slip-Stick-Effekte eine stufenlose und kraftkonstante Verlagerung der Segmente oder Lamellen vereiteln können, sondern eine gleitreibungsfreie Festkörpergelenklagerung realisiert wird.

Ebenfalls beispielhaft und besonders vorteilhaft ist eine bewegliche Lagerung entlang des Doppelpfeils 424 des Anpressstempels in Z-Richtung relativ zur Aktor-Sensor-Platte 290. Auf diese Weise kann beispielsweise eine Feder oder ein Druckluftaktor den gesamten Stempel mit einer konstanten Kraft belasten, die der erforderlichen Gesamt-Verpresskraft für die Schließstellung des Ventils 288 entspricht und ein weiteres Element des Toleranz-, Relaxations- und Abnützungs-Ausgleichs ist. Da man je nach Lagerungsart der Feder und nach Betriebsart eines Stempel-Aktors diese Kraft auch abhängig von der Aufrüststellung der Kassette 1000 abschalten kann bzw. den möglichen Weg beschränken kann, wird zugleich eine Schonung des Materials des Aktor-Sensor-Matte 291 für die Betriebszustände bei nicht aufgerüsteter Kassette 1000 erreicht.

Eine ähnliche Wirkung wie die beispielhaft dargestellten Lamellenfedern auf Biegegelenkbasis können Anpressstempel entfalten, die ähnlich dem dargestellten Stempel segmentiert sind und als federnde Elemente beispielsweise eine elastische Schicht aus, vorzugsweise geschlossenzelligem, Schaumstoff erhalten. In manchen erfindungsgemäßen Ausführungsformen können die Lamellen sogar entfallen, wobei der Schaumstoff direkt zwischen Aktor-Sensor-Matte 291 und Anpressstempel angeordnet wird. Der bewegliche Anpressstempel dient dann der Erzeugung der definierten globalen Ventil-Anpresskraft und dem zuletzt beschriebenen Toleranzausgleich.

Eine ähnliche Wirkung kann ein statt des Schaumstoffes zwischen Aktor-Sensor-Matte 291 und Anpressstempel eingefügtes fluiddichtes Kissen aus Elastomer-Material mit Gas-Füllung oder Gel-Füllung entfalten. Eine weitere Lösungsmöglichkeit, ähnlich dem oben beschriebenen Lamellen- Anpressstempel, kann beispielweise auch ein Strangpressprofil aus einem Elastomer entfalten, das auf den Stirnflächen Rippen ähnlich den obengenannten Lamellen aufweist und im Inneren parallel zu den Rippen und beispielsweise zentrisch unter jeder Rippe durchlaufende Hohlprofile aufweist. Sieht man anstelle von Rippen kurze abgesetzte Rippenstücke oder einzelne Höcker, beispielsweise quadratische Höcker vor, so erhält man einen toleranzausgleichenden Anpressstempel, welcher seine toleranzausgleichende Wirkung nicht nur in eine Raumrichtung wie die obengenannte Raumrichtung längs der Dichtsitzkante 226, sondern in beide Raumrichtungen entfaltet. Dies kann vorteilhaft sein für Folienventile 288 mit zugleich langen und breiten Dichtsitzbereichen 226, wie sie beispielsweise bei der Verbindung weiter voneinander entfernter Kammern 202 und Kanäle 201 vorkommen können.

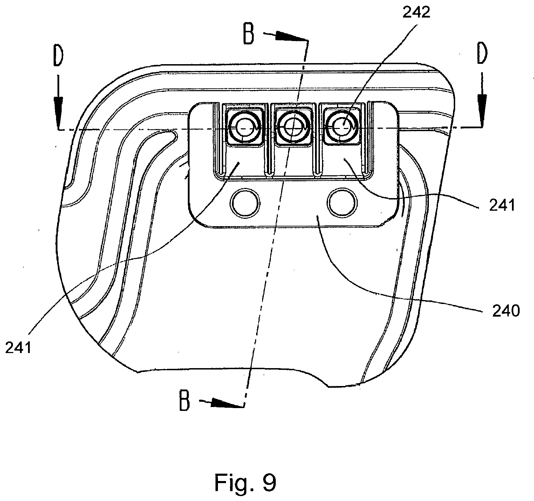

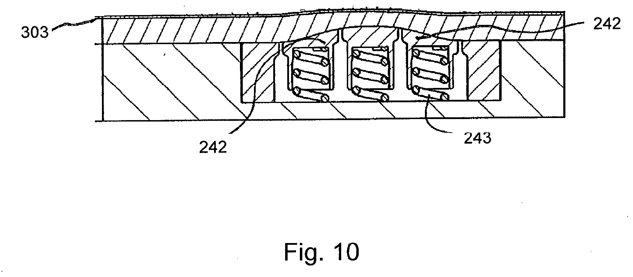

**Fig.** 9 und **Fig. 10** zeigen eine Ausführungsform einer in die Blutbehandlungsvorrichtung 5000 eingelegten und von dieser verpressten Kassette 1000, also im aufgerüsteten Zustand, in Draufsicht (Fig. 9) und in Teil-Schnittansicht entlang der Linie D-D der Fig. 9 (in Fig. 10) mit horizontal ausgerichteter Folienebene.

In Fig. 9 ist von der Blutbehandlungsvorrichtung 5000 nur ein Anpressstempel 240 als Teil einer Aktor-Sensor-Platte 290 zu sehen. Der Anpressstempel 240 ist ein Beispiel eines Aktuators. In dieser beispielhaften erfindungsgemäßen Ausführungsform ist der Anpressstempel 240 in Lamellenstempel 242 (Teil-Aktuatoren) aufgeteilt, siehe die Fig. 10, die über Biegegelenke 241 an den Grundkörper des Anpressstempels 240 angebunden sind.

Hierbei ergeben sich ein Einbau-Toleranz-Ausgleich zwischen Kassette 1000 und Blutbehandlungsvorrichtung 5000. Ferner verteilt sich die Ventil-Dichtpresskraft im Gebrauch abnützungsresistent und gleichmäßig auf den gesamten gedellten Ventilgrund. Zur relaxationsfreien Krafterzeugung sind hier beispielhaft Spiralfedern 243 aus Federstahl vorgesehen, die sich in einfacher Weise bei der Montage des Anpressstempels 240 in eine Tasche der Aktor-Sensor-Platte 290 zwischenfügen und vorspannen lassen. Der komplette Anpressstempel 240 kann preiswert aus unverstärkten oder faserverstärkten Thermoplasten in Auf-Zu-Spritzgießwerkzeugen hergestellt werden.

**Bezugszeichenliste**

| **Bezugszeichen** | **Beschreibung** |
|---|---|
| 1000 | Kassette |
| 1 | Hartteil |
| 3 | Folie |
| 4 | Dichtsteg |
| 5 | umlaufende Schweißnaht |
| 9 | arterielle Druckmesskammer |
| 11 | Konnektor für Blutaustritt aus Kassette 1000 |
| 13 | Konnektor für Bluteintritt in Kassette 1000 |
| 15 | Kammer mit arterieller Post-Pumpe- bzw. PräFilter-Druckmessstelle |
| 17 | arterielle Filterleitung |
| 19 | venöse Filterleitung |
| 21 | venöse Blutkammer |
| 23 | oberer Raum der venösen Blutkammer 21 |
| 25 | unterer Raum der venösen Blutkammer 21 |
| 27 | Querschnittsverjüngung des Hartteils 1 |
| 29 | Gerinnselfänger |
| 31 | venöser Patientenanschluss |
| 33 | arterielle Heparin-Zugabestelle |
| 35 | Rückschlagventil der arteriellen Heparin-Zugabestelle 33 |
| 36 | arterielles Heparin-Zugabeventil (Phantomventil) |
| 37 | venöse Heparin-Zugabestelle |
| 39 | Rückschlagventil der venösen Heparin-Zugabestelle 37 |
| 40 | venöses Heparin-Zugabeventil (Phantomventil) |
| 41 | Substituat-Zugabestelle |
| 43 | Konnektor für Substituataustritt aus der Kassette 1000 |
| 45 | Konnektor für Substituateintritt in die Kassette 1000 |
| 47 | Rückschlagventil für Substituatzugabe |
| 49 | Substituatleitung |
| 51 | Prädilutions-Zugabeventil (Phantomventil) |
| 53 | Postdilutions-Zugabeventil (Phantomventil) |
| 55 | Single-Needle-Sterilmembran |
| 57 | Single-Needle-Kammer |
| 59 | Blutschwallumleitungselement |
| 61 | Single-Needle-Blutventil (Phantomventil) |
| 63 | Absaugstelle für Vakuumankopplung |
| 65 | primäres Ausrichtungszentrum |
| 67 | sekundäre Ausrichtungsstelle |
| 69 | Dichtsteg |
| 71 | Single-Needle-Luft-Konnektor |
| 73 | Stützstege mit 5 mm Höhe |
| 75 | Stützstege mit 8 mm Höhe |
| 77 | Stützstege mit 13 mm Höhe |
| 79 | Stützstege mit 24 mm Höhe |
| 81 | Stützstege mit 31 mm Höhe |
| 201 | Kanal |
| 202 | Kammer |
| 204 | umlaufende ebene Kanalrandstege |
| 226 | Abschnitt oder Foliendichtsitz-Steg oder Ventilgrund |
| 240 | Aktuator |
| 241 | Aktuator, Biegegelenke |
| 242 | Teil-Aktuator |
| 243 | Spiralfeder |
| 288 | Phantom- oder Folienventil |
| 290 | Aktor-Sensor-Platte |
| 418 | Erhebung oder Ausbeulung |
| 419 | dünnerer Abschnitt |
| 420 | Stempelkörper |
| 421 | Lamellenstempel |
| 422 | Lamellenfeder |
| 424 | Z-Richtung, Doppelpfeil |
| 5000 | Blutbehandlungsvorrichtung |

## Patentansprüche

1. Blutbehandlungskassette (1000) mit einem als Hartteil (1) ausgestalteten Kassettenkörper und genau einer Folie (3), wobei die Folie (3) mit dem Hartteil (1) verbunden ist und das Hartteil (1) zumindest abschnittsweise abdeckt,
wobei das Hartteil (1) wenigstens einen gedellten Ventilgrund (226), welcher konkav oder konvex ist, eines Phantomventils (288) aufweist, wobei das Ventil (288) ausgestaltet ist, um neben einer ersten, geöffneten Stellung des Ventils (288), in welcher der Ventilgrund (226) und ein über diesem angeordneter Abschnitt der Folie (3) einander nicht berühren, bei Aufbringen einer Kraft auf einen Abschnitt der Folie (3) eine zweite, geschlossene Stellung des Ventils (288) einzunehmen, in welcher der Ventilgrund (226) und der Abschnitt der Folie (3) einander berühren,
wobei der Ventilgrund (226) in seiner Längserstreckung einen nicht-geraden Abschnitt aufweist oder hieraus besteht und
wobei eine Dellentiefe (T) des Ventilgrunds (226) dem 1-bis 3-fachen der Dicke oder Stärke der Folie (3) entspricht oder der Ventilgrund (226) hinter benachbarten Kanalrandstegen (204) um das 1- bis 3-fache der Dicke oder Stärke der Folie (3) in Richtung Inneres der Kassette (1000) zurückgesetzt ist,
wobei die Blutbehandlungskassette
(1000) einen Konnektor (11) für den Blutaustritt aus der Kassette (1000) sowie einen Konnektor (13) für den Bluteintritt in die Kassette (1000) aufweist, wobei die beiden Konnektoren (11) und (13) mit einem Pumpschlauchsegment oder Pumpschlauchset einer Blutpumpe verbindbar sind,
wobei die Blutbehandlungskassette (1000) einen Konnektor (43) für einen Substituataustritt aus der Kassette (1000) sowie einen Konnektor (45) für einen Substituateintritt in die Blutbehandlungskassette (1000) aufweist, wobei die Konnektoren (43) und (45) mit einem Pumpschlauchsegment oder Pumpschlauchset einer Substituatpumpe verbindbar sind.

2. Blutbehandlungskassette (1000) nach Anspruch 1, wobei das Ventil (288) ausgestaltet ist, um mittels Druckaufbringung eines Aktuators (240, 241, 242) einer Blutbehandlungsvorrichtung (5000) auf das Ventil (288), zu deren Betrieb die Blutbehandlungskassette (1000) bestimmungsgemäß mit der Blutbehandlungsvorrichtung (5000) verbunden wird, von der ersten Stellung in die zweite Stellung überführbar zu sein.

3. Blutbehandlungskassette (1000) nach einem der vorangegangenen Ansprüche, wobei die Dellentiefe (T) stetig zu- und/oder abnimmt.

4. Blutbehandlungsvorrichtung (5000), verbunden mit einer Blutbehandlungskassette (1000) nach einem der Ansprüche 1 bis 3 oder zur Verbindung hiermit vorgesehen, konfiguriert oder geeignet, mit einer Aktor-Sensor-Platte (290), wobei die Aktor-Sensor-Platte (290) wenigstens einen Aktuator (240) aufweist, welcher wenigstens zwei, vorzugsweise mehr als drei, Teil-Aktuatoren (242) aufweist, welche angeordnet sind, um unabhängig voneinander Kraft auf den Abschnitt der Folie (3) des Ventils auszuüben..

5. Blutbehandlungsvorrichtung (5000) nach Anspruch 4, wobei die Aktor-Sensor-Platte (290) eine der Blutbehandlungskassette (1000) zugewandte Aktor-Sensor-Matte (291) aufweist, welche einen Abschnitt (418) aufweist, welcher dünner ist als benachbarte Abschnitte, und welcher dem Ventilgrund (226) der Blutbehandlungskassette (1000) zugewandt ist.

## Claims

1. A blood treatment cassette (1000) comprising a cassette body designed as a hard part (1) and exactly one film (3), the film (3) being connected with the hard part (1) and covering the hard part (1) at least in sections,
wherein the hard part (1) comprises at least one concave or convex depressed valve base (226) of a phantom valve (288), the valve (288) being designed to take,
in addition to a first, open position of the valve (288), in which the valve base (226) and a portion of the film (3) disposed above it don't touch each other,
a second, closed position of the valve (288), in which the valve base (226) and the portion of the film (3) touch each other when a force is applied to a portion of the film (3),
wherein the valve base (226) comprises a non-straight portion in its longitudinal extension or consists thereof and
wherein a depression depth (T) of the valve base (226) corresponds to 1 to 3 times the thickness or strength of the film (3) or the valve base (226) is set back behind adjacent channel border bridges (204) up to 1 to 3 times the thickness or strength of the film (3) towards the interior of the cassette (1000),
wherein the blood treatment cassette (1000) comprises a connector (11) for the blood outlet from the cassette (1000) as well as a connector (13) for the blood inlet into the cassette (1000), the two connectors (11, 13) being connectable with a pump tube segment or a pump tubing set of a blood pump,
wherein the blood treatment cassette (1000) comprises a connector (43) for a substitute outlet from the cassette (1000) as well as a connector (45) for a substitute inlet into the blood treatment cassette (1000), the connectors (43, 45) being connectable with a pump tube segment or a pump tubing set of a substitute pump.

2. The blood treatment cassette (1000) according to claim 1, wherein the valve (288) is designed to be transferable from the first to the second position by applying a pressure with an actuator (240, 241, 242) of a blood treatment apparatus (5000) to the valve (288), for the operation of which the blood treatment cassette (1000) is as intended connected to the blood treatment apparatus (5000).

3. The blood treatment cassette (1000) according to anyone of the preceding claims, wherein the depression depth (T) steadily increases and/or decreases.

4. A blood treatment apparatus (5000), connected with a blood treatment cassette (1000) according to anyone of claims 1 to 3, or intended, configured or suitable for such a connection, comprising an actuator sensor plate (290), said actuator sensor plate (290) having at least one actuator (240) having at least two, preferably more than three, partial actuators (242) arranged to exert a force on the portion of the film (3) of the valve independently of each other.

5. The blood treatment apparatus (5000) according to claim 4, wherein the actuator sensor plate (290) comprises an actuator sensor mat (291) facing the blood treatment cassette (1000), which has a thinner portion (418) than adjacent portions and which faces the valve base (226) of the blood treatment cassette (1000).

## Revendications

1. Une cassette de traitement du sang (1000) comprenant un corps de cassette conçu comme une partie rigide (1) et exactement un film (3), le film (3) étant relié à la partie rigide (1) et recouvrant la partie rigide (1) au moins par sections,
où la partie rigide (1) comprend au moins un fond de vanne (226) enfoncé, concave ou convexe, d'une vanne fantôme (288), la vanne (288) étant conçue pour prendre, en plus d'une première position ouverte de la vanne (288), dans laquelle le fond de vanne (226) et une section du film (3) disposée au-dessus de celui-ci ne se touchent pas, une seconde position fermée de la vanne (288), dans laquelle le fond de vanne (226) et la section du film (3) se touchent lorsqu'une force est appliquée sur une section du film (3),
où le fond de vanne (226) comprend une section non linéaire dans son extension longitudinale ou est constitué de celle-ci et
où une profondeur d'enfoncement (T) du fond de vanne (226) correspond à 1 à 3 fois l'épaisseur ou la hauteur du film (3) ou où le fond de vanne (226) est décalé de 1 à 3 fois l'épaisseur ou la hauteur du film (3) vers l'intérieur de la cassette (1000) à l'arrière de bords de traverses de conduits (204) adjacents,
où la cassette de traitement du sang (1000) comprend un connecteur (11) pour l'évacuation du sang de la cassette (1000) ainsi qu'un connecteur (13) pour l'introduction du sang dans la cassette (1000), les deux connecteurs (11, 13) pouvant être reliés à un segment de tuyaux de pompe ou à un set de tuyaux de pompe d'une pompe à sang,
où la cassette de traitement du sang (1000) comprend un connecteur (43) pour une évacuation du substitut de la cassette (1000) ainsi qu'un connecteur (45) pour une introduction du substitut dans la cassette de traitement du sang (1000), les connecteurs (43, 45) pouvant être reliés à un segment de tuyaux de pompe ou à un set de tuyaux de pompe d'une pompe à substitut.

2. La cassette de traitement du sang (1000) selon la première revendication, où la vanne (288) est conçue pour être transférable de la première position à la seconde position par application d'une pression d'un actionneur (240, 241, 242) d'un appareil de traitement du sang (5000) sur la vanne (288), pour le fonctionnement duquel la cassette de traitement du sang (1000) est reliée à l'appareil de traitement du sang (5000) conformément à son usage prévu.

3. La cassette de traitement du sang (1000) selon l'une quelconque des revendications précédentes où la profondeur d'enfoncement (T) augmente et/ou diminue progressivement.

4. Un appareil de traitement du sang (5000), relié à une cassette de traitement du sang (1000) selon l'une quelconque des revendications 1 à 3, ou prévu, configuré ou adapté pour y être relié,
comprenant une plaque actionneur-capteur (290), ladite plaque actionneur-capteur (290) comprenant au moins un actionneur (240) ayant au moins deux, de préférence plus de trois, actionneurs partiels (242) agencés de façon à pouvoir exercer, indépendamment les uns des autres, une force sur la section du film (3) de la vanne.

5. L'appareil de traitement du sang (5000) selon la revendication 4, où la plaque actionneur-capteur (290) comprend un tapis actionneur-capteur (291) faisant face à la cassette de traitement du sang (1000)
ayant une section (418) plus mince que les sections adjacentes et faisant face au fond de vanne (226) de la cassette de traitement du sang (1000).
